# EUROPEAN PATENT SPECIFICATION

(11) **EP 4 023 471 B1**
(45) Date of publication and mention of the grant of the patent: **17.09.2025**
(21) Application number: 20864561.4
(22) Date of filing: 17.09.2020
(51) Int. Cl.: B60H 3/00, B60H 1/00, A61L 9/20, B60N 2/56

(54) **DUCT MODULE AND VEHICLE SEAT INCLUDING DUCT MODULE**
KANALMODUL UND FAHRZEUGSITZ MIT KANALMODUL
MODULE DE CONDUIT ET SIÈGE DE VÉHICULE COMPRENANT LE MODULE DE CONDUIT

(30) Priority: 19.09.2019 KR 20190115595
(43) Date of publication of application: 06.07.2022
(73) Proprietor: Seoul Semiconductor Co., Ltd., Gyeonggi-do 15429 (KR)
(72) Inventor: PARK, In Heum, Ansan-Si Gyeonggi-do 15429 (KR); KIM, Jiwon, Gyeonggi-do 15429 (KR); SHIN, Sangchul, Gyeonggi-do 15429 (KR); JEONG, Jaehak, Gyeonggi-do 15429 (KR)
(74) Representative: Isarpatent
(86) International application number: PCT/KR2020/012568
(87) International publication number: WO 2021/054743

(56) References cited:
- DE-T5- 112015 000 091
- JP-A- 2017 503 708
- KR-A- 20070 079 108
- KR-B1- 100 754 014
- US-A1- 2004 071 589
- US-A1- 2004 071 589
- US-A1- 2014 179 212
- US-A1- 2018 056 758

## Description

### [Technical Field]

The present invention relates to a duct module and a vehicle seat including the duct module.

### [Background Art]

In general, a vehicle ventilates internal air of a vehicle using an air conditioner. The air conditioner ventilates the air inside the vehicle by intaking in the external air of the vehicle and discharging the intaken air into the interior of the vehicle.

In this case, if the air outside the vehicle contains contaminants such as dust and bacteria, the air conditioner may discharge the polluted air into the inside of the vehicle as it is.

In order to circulate clean air inside a vehicle, in the related art, an air purification device is installed in an air conditioner. Accordingly, the external air is purified, while passing through the air conditioner, and the air conditioner discharges purified air into the vehicle.

However, when the air purification device is installed in the air conditioner, only contaminants introduced through the air conditioner are removed. That is, in the related art, it is impossible to remove contaminants introduced into the vehicle through a path other than the air conditioner.

Therefore, there is a limitation in purifying the air inside the vehicle by removing contaminants inside the vehicle with the conventional air conditioner equipped with an air purifier.

US 2004/071589 A1 provides an apparatus for decontaminating air within an enclosed workspace located downstream and in fluid communication with the apparatus. US 2014/179212 A1 provides a method for providing purified air to an occupant of a seat in a mask lacking corporeal features that is generated by an apparatus.

### [Disclosure]

### [Technical Problem]

The present invention provides a duct module capable of purifying air inside a vehicle by sterilizing contaminants inside the vehicle, and a vehicle seat including the duct module.

The present invention provides a duct module for sterilizing contaminants inside a vehicle, which may be easily installed anywhere in the vehicle.

### [Technical Solution]

According to the present invention, a duct module including a duct, an air intake module and a sterilization module is provided, as defined by independent claim 1.

According to the present invention, a duct module is provided, as defined by independent claim 10.

The duct air passage is formed as an internal space of the first rigid portion, the second rigid portion, and the flexible portion.

### [Advantageous Effects]

According to an exemplary embodiment of the present invention, since the duct module sterilizes contaminants by intaking air inside the vehicle, an air purification rate inside the vehicle may be improved.

According to an exemplary embodiment of the present invention, the duct module may sterilize contaminants introduced into the vehicle, regardless of an inflow path of contaminants.

According to an exemplary embodiment of the present invention, since it is easy to change a structure of the duct, the duct module may be installed anywhere, regardless of an internal structure of the vehicle.

According to an exemplary embodiment of the present invention, since sterilized air is discharged through the through-holes of the seat in the duct module of the vehicle seat, a surface of the seat may be maintained to be clean.

### [BRIEF DESCRIPTION OF THE DRAWINGS]

FIGS. 1 and 2 are exemplary views illustrating a vehicle seat according to an exemplary embodiment of the present invention.
FIG. 3 is an exemplary view illustrating a duct module according to the first exemplary embodiment.
FIG. 4 is an exemplary view illustrating a duct module according to a second exemplary embodiment of the present invention.
FIG. 5 is an exemplary view illustrating a duct module according to a third exemplary embodiment of the present invention.
FIG. 6 is an exemplary view illustrating a duct module according to a fourth exemplary embodiment of the present invention.
FIG. 7 is an exemplary view illustrating a duct module according to a fifth exemplary embodiment of the present invention.
FIG. 8 is an exemplary view illustrating a duct module according to a sixth exemplary embodiment of the present invention.
FIG. 9 is an exemplary view illustrating a duct module according to a seventh exemplary embodiment of the present invention.
FIG. 10 is an exemplary view illustrating a duct module according to an eighth exemplary embodiment of the present invention.
FIGS. 11 and 12 are exemplary views illustrating a duct module according to a ninth exemplary embodiment of the present invention.
FIG. 13 is an exemplary view illustrating a duct module according to a tenth exemplary embodiment of the present invention.
FIG. 14 is an exemplary view illustrating a duct module according to an eleventh exemplary embodiment of the present invention.

### [Best Model

Hereinafter, exemplary embodiments of the present invention will be described in detail with reference to the accompanying drawings. The exemplary embodiments of the present invention to be introduced below are provided by way of example so that the idea of the present invention can be sufficiently transferred to those skilled in the art to which the present invention pertains. In the drawings, the sizes, lengths, and thicknesses of elements may be exaggerated for convenience of explanation. Throughout the specification, like reference numerals denotes like components.

According to an exemplary embodiment of the present invention, a duct module includes a duct, an air intake module and a sterilization module. The duct may have a duct air passage formed therein and include a first rigid portion, a second rigid portion, and a flexible portion disposed between the first rigid portion and the second rigid portion. The air intake module may be connected to the first rigid portion of the duct and guide external air into the duct air passage. Further, the sterilization module may be disposed in the duct to emit sterilization light into the duct air passage. The duct air passage may be formed as an internal space of the first rigid portion, the second rigid portion and the flexible portion.

The sterilization light may be ultraviolet light.

The sterilization module includes a substrate and a sterilization light source. The sterilization light source may be mounted on the substrate and emit the sterilization light.

The sterilization module may be disposed on an inner side surface of the second rigid portion of the duct.

The sterilization module may be disposed to penetrate through a side surface of the duct.

The sterilization module may further include a sterilization module body having one side and the other side open and having an internal space connecting the one side and the other side. In this case, the substrate and the sterilization light source may be disposed in the internal space of the sterilization module body.

The one side of the sterilization module body may be connected to the flexible portion, and the other side may be connected to the second rigid portion.

The flexible portion may include a first flexible portion connected to the first rigid portion and a second flexible portion connected to the second rigid portion. In this case, the one side of the sterilization module body may be connected to the first flexible portion, and the other side may be connected to the second flexible portion.

The sterilization module may be disposed in plurality in the duct.

The flexible portion of the duct may have a variable length.

The air intake module may include a fan. An air outlet of the fan may be connected to the first rigid portion of the duct.

A vehicle seat according to an exemplary embodiment of the present invention may include a seat and a duct module.

The seat may include a seat portion and a backrest portion having a breathable structure in which a plurality of through-holes are formed on a surface thereof. In addition, the seat may have a seat air passage connected to the plurality of through-holes therein.

The duct module may be disposed on one side of the seat to sterilize the air inside the vehicle. Also, the duct module may discharge the sterilized air to the seat air passage of the seat.

The duct module may include a duct, an air intake module, and a sterilization module. The duct may have a duct air passage therein and include a first rigid portion, a second rigid portion, and a flexible portion disposed between the first rigid portion and the second rigid portion. The air intake module may be connected to the first rigid portion of the duct and guide air inside the vehicle to the duct air passage. Further, the sterilization module may be disposed in the duct to discharge sterilization light into the duct air passage. The duct air passage may be formed as an internal space of the first rigid portion, the second rigid portion, and the flexible portion.

The seat air passage of the seat may be connected to the second rigid portion of the duct.

The sterilization light may be ultraviolet light.

The sterilization module may include a substrate and a sterilization light source. The sterilization light source may be mounted on the substrate and emit the sterilization light.

The sterilization module may be disposed on the inner side surface of the second rigid portion of the duct.

The sterilization module may be disposed to penetrate through a side surface of the duct.

The sterilization module may further include a sterilization module body having one side and the other side open and having an internal space connecting the one side and the other side. The substrate and the sterilization light source may be disposed in the internal space of the sterilization module body.

The one side of the sterilization module body may be connected to the flexible portion, and the other side may be connected to the second rigid portion.

The flexible portion may include a first flexible portion connected to the first rigid portion and a second flexible portion connected to the second rigid portion. In this case, the one side of the sterilization module body may be connected to the first flexible portion, and the other side may be connected to the second flexible portion.

The sterilization module may be disposed in plurality in the duct.

The flexible portion of the duct may have a variable length.

The air intake module may include a fan. In this case, an air outlet of the fan may be connected to the first rigid portion of the duct.

Hereinafter, a light treatment device of the present invention will be described in detail with reference to the drawings.

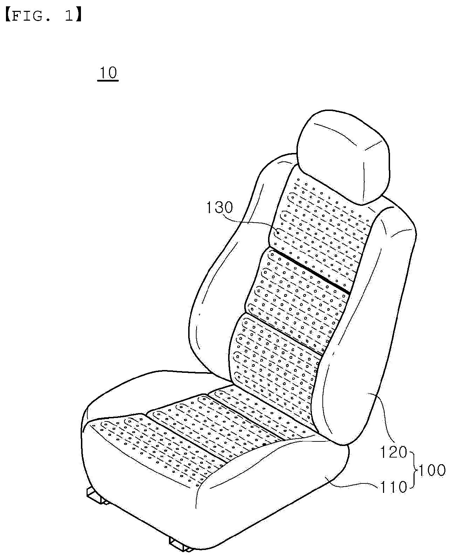

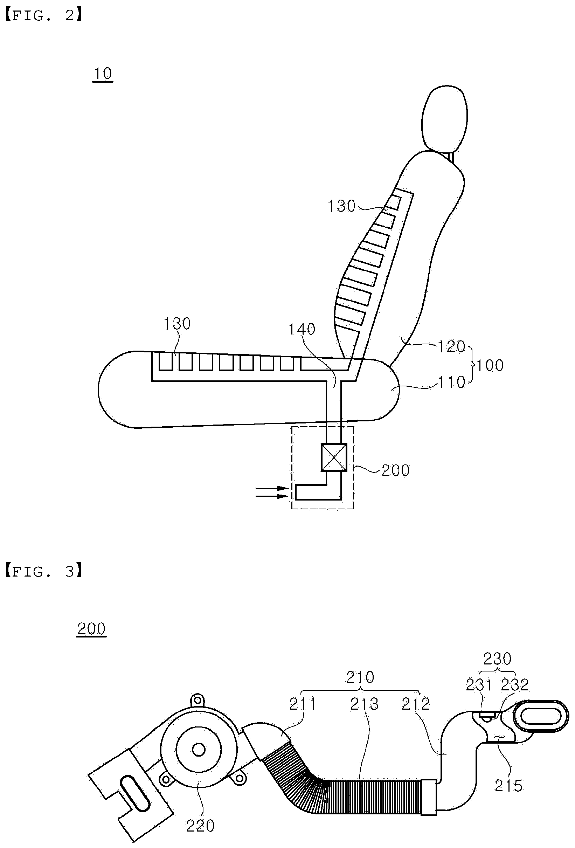

FIGS. 1 and 2 are exemplary views illustrating a vehicle seat according to an exemplary embodiment of the present invention. FIG. 1 is an exemplary view illustrating a front side of a vehicle seat 10 of the present exemplary embodiment. FIG. 2 is an exemplary view schematically illustrating a side cross-section of the vehicle seat 10 according to an exemplary embodiment of the present invention.

In addition, FIG. 3 is an exemplary view illustrating a duct module according to the first exemplary embodiment.

According to the present exemplary embodiment, the vehicle seat 10 includes a seat 100 and a duct module 200.

The seat 100 disposed in the interior of a vehicle is divided into a seat portion 110 and a backrest portion 120.

The seat portion 110 and the backrest portion 120 have a breathable structure in which a plurality of through-holes 130 are formed on a front surface as shown in FIGS. 1 and 2.

In addition, a seat air passage 140 through which air may flow is formed inside the seat 100. An inlet of the seat air passage 140, through which air is introduced, is connected to the duct module 200 disposed outside the seat 100. In addition, an outlet of the seat air passage 140, through which air is discharged, is connected to the through-hole 130 of the seat 100.

Accordingly, the air introduced into the seat 100 through the duct module 200 moves through the seat air passage 140 and is discharged to the outside through the through-hole 130 of the seat 100.

Referring to FIG. 2, the duct module 200 is disposed on one side of the seat 100 and is connected to the seat air passage 140 of the seat 100.

Referring to FIG. 3, the duct module 200 includes a duct 210, an air intake module 220, and a sterilization module 230.

The duct 210 includes a first rigid portion 211, a second rigid portion 212, and a flexible portion 213. The first rigid portion 211, the second rigid portion 212, and the flexible portion 213 are connected to each other in an internal space. That is, the internal space of the first rigid portion 211, the second rigid portion 212, and the flexible portion 213 becomes the duct air passage 215, which is a passage through which air moves.

One end of the first rigid portion 211 is connected to the air intake module 220, and the other end is connected to the flexible portion 213. The first rigid portion 211 may not be changed in shape by an external force, and thus may be stably connected to the air intake module 220 and the flexible portion 213.

One end of the second rigid portion 212 is connected to the flexible portion 213 and the other end thereof is connected to the seat air passage 140 of the seat 100. Like the first rigid portion 212, the second rigid portion 212 may not be changed in shape by an external force, and thus may be stably connected to the flexible portion 213 and the seat air passage 140.

The flexible portion 213 is disposed between the first rigid portion 211 and the second rigid portion 212. One end of the rigid portion is connected to the first rigid portion 211, and the other end is connected to the second rigid portion 212.

In addition, the flexible portion 213 is formed of a material or structure that may be easily bent. Accordingly, the duct module 200 may be easily installed on the seat 100 by the flexible portion 213, regardless of a structure of an installation site.

In addition, as shown in FIG. 3, in the flexible portion 213, a side surface surrounding the internal space in which air moves has a creased structure. In addition, the crease of the flexible portion 213 may be stretched or compressed. Accordingly, a length of the flexible portion 213 may be increased or decreased.

Therefore, even when the position of the seat 100 is changed, the flexible portion 213 is bent or the length is changed, and thus, it is possible to prevent the coupling of the duct module 200 and the seat 100 from being separated.

The air intake module 220 has an inlet for intaking air at one end thereof and an outlet for discharging air at the other end thereof. The other end of the air intake module 220, which is an outlet, is connected to the first rigid portion 211 of the duct 210. Accordingly, the air intake module 220 intakes the air inside the vehicle and discharges it to the duct air passage 215 of the duct 210.

According to the present exemplary embodiment, the air intake module 220 includes a fan disposed therein. In the air intake module 220, external air is intaken in by the fan, and the intaken air is discharged to the duct 210. Here, the external air of the air intake module 220 is internal air of the vehicle.

In the present exemplary embodiment, the fan is one of the components constituting the air intake module 220. However, a structure of the air intake module 220 is not limited thereto. The fan itself may be an air intake module 220. In addition, the air intake module 220 may have any structure as long as the structure may discharge the intaken air to the duct 210.

The sterilization module 230 may be disposed in the duct 210 to emit sterilization light to the duct air passage 215. That is, the sterilization module 230 sterilizes the air flowing through the duct air passage 215.

According to the present exemplary embodiment, the sterilization module 230 is fixed to an inner side surface of the second rigid portion 212.

The sterilization module 230 includes a substrate 231 and a sterilization light source 232.

The sterilization light source 232 is mounted on the substrate 231 and is electrically connected to the substrate 231. The sterilization light source 232 receives power through the substrate 231 and emits sterilization light. For example, the sterilization light source 232 may be a light emitting device (LED). In addition, the sterilization light may be ultraviolet rays in a UVC wavelength band. However, the type of sterilization light is not limited to UVC. The sterilization light may be any kind of light as long as it has a sterilizing function.

In addition, the sterilization light source 232 may have a beam angle of 90° to 140°.

The substrate 231 may include a wiring board on which a wiring electrically connected to the sterilization light source 232 is formed. Further, the substrate 231 may include a fixed substrate fixed to the duct 210 such that the sterilization light source 232 faces the duct air passage 215. Alternatively, the substrate 231 may be formed of only a wiring board, and the wiring board may be fixed to the duct 210.

A connector (not shown) connected to a wiring may be formed on the substrate 231. Depending on a position of the substrate 231, the connector may be connected to an electric wire connected to an external power device inside or outside the duct 210. A method for the sterilization light source 232 to receive power is not limited thereto, and the sterilization light source 232 may receive power according to various known methods.

According to the present exemplary embodiment, the sterilization module 230 is disposed such that the sterilization light source 232 is mounted on one surface of the substrate 231 and the other surface of the substrate 231 is in contact with an inner side surface of the second rigid portion 212. Accordingly, the sterilization module 230 irradiates sterilization light toward the air passing through a region in which the sterilization module 230 is disposed.

In order for the sterilization module 230 to be disposed in front of the inlet of the air intake module 220, a separate component for fixing the sterilization module 230 in front of the inlet of the air intake module 220 is required. However, in the duct module 200 of the present exemplary embodiment, since the sterilization module 230 is fixed inside the duct 210, a separate component for fixing the sterilization module 230 to the duct module 200 may be omitted.

According to an exemplary embodiment of the present invention, the duct module 200 intakes air inside the vehicle into the duct 210 through the air intake module 220. The intaken air is sterilized by the sterilization module 230, while moving along an internal passage of the duct 210. In addition, the duct module 200 discharges sterilized air to the seat air passage 140 of the seat 100.

The sterilized air discharged to the seat air passage 140 through the duct module 200 is discharged to the outside of the seat 100 through the through-hole 130 formed on the surface of the seat 100.

That is, according to an exemplary embodiment of the present invention, the vehicle seat 10 including the duct module 200 and the seat 100 intakes and sterilizes the air inside the vehicle, and then discharges the sterilized air back into the vehicle.

Air pollution inside a vehicle may occur when polluted air from the outside flows into the interior. In addition, the air pollution inside the vehicle is not caused by the inflow of air from the outside of the vehicle, but also occurs inside the vehicle. For example, external polluted air may be introduced into the vehicle through an air conditioner of the vehicle. In addition, external air may be introduced into the interior of the vehicle through a window or door of the vehicle. In addition, the air of the vehicle may be contaminated by external contaminants attached to the body of an occupant. For example, the contaminant may be dust, pollen, germs such as bacteria, and the like.

A vehicle air purification module of the related art is installed in an air conditioner for heating, ventilation, air conditioning, and the like of a vehicle. An air conditioner is a device that introduces air from the outside of the vehicle into the interior of the vehicle. That is, the vehicle air purification system of the related art purifies external air introduced through the air conditioner.

Accordingly, it is difficult to purify the air introduced into the vehicle through a window or door or to sterilize contaminants occurring by an occupant using the vehicle air purification system of the related art.

In addition, the vehicle air purification system of the related art cannot sterilize contaminants occurring in the interior space of the vehicle. Accordingly, in order to sterilize contaminants occurring in the vehicle interior space, a separate sterilization device should be installed in the vehicle interior space. However, since sterilization light emitted from the sterilization device is in an ultraviolet wavelength band, there is a problem that it is harmful to the human body.

In addition, the air conditioner has a complex internal space and a large cross-sectional area. Therefore, it is difficult for the ultraviolet rays of the air purification module to be irradiated to the entire interior space of the air conditioner. That is, it is difficult to sufficiently sterilize the external air passing through the internal space of the air conditioner with the air purification system of the related art. In order to sufficiently sterilize the external air, a large number of air purification modules should be distributed and installed inside the air conditioner.

The vehicle seat 10 of the present exemplary embodiment is sterilized by intaking air inside the vehicle. Accordingly, the vehicle seat 10 of the present exemplary embodiment may purify air introduced into the vehicle via other portions such as windows and doors as well as the air conditioner. In addition, the vehicle seat 10 of the present exemplary embodiment may purify the air inside the vehicle contaminated by contaminants transferred into the vehicle through the occupant.

As described above, since the vehicle seat 10 according to the present exemplary embodiment intakes and sterilizes the air inside the vehicle, regardless of an inflow path of the contaminants, the air purification effect inside the vehicle may be improved.

In addition, in the vehicle seat 10 according to the present exemplary embodiment, the contaminants inside the vehicle are sterilized inside the duct 210. Accordingly, it is possible to prevent contaminants from accumulating inside the duct 210. In addition, since sterilization light of the sterilization module 230 is not emitted to the outside of the duct 210 in the vehicle seat 10 of the present exemplary embodiment, a risk of exposing a human body of the vehicle occupant to UV rays may be prevented.

A lot of contaminants by the occupant may be attached on the surface of the seat 100 of the vehicle. In the vehicle seat 10 according to the present exemplary embodiment, since sterilized air is discharged through the surface of the seat 100, an effect of removing contaminants attached to the surface of the seat 100 may be obtained. That is, the vehicle seat 100 of the present exemplary embodiment may keep the surface of the seat 100 clean. At this time, the contaminants separated from the surface of the seat 100 may be intaken through the duct module 200 and sterilized.

The duct module 200 of the present exemplary embodiment may further include a controller (not shown). The controller may control the operation of the duct module 200 according to a preset value. For example, the controller may control the operations of the air intake module 220 and the sterilization module 230 so that the air sterilization operation is performed for a preset time and then stopped when the vehicle is turned off.

Hereinafter, various exemplary embodiments of the duct module will be described. When describing the exemplary embodiment of the duct module, a difference from the duct module 200 of the first exemplary embodiment will be mainly described. For the omitted components, the descriptions of the duct module 200 of the first exemplary embodiment may be referred to.

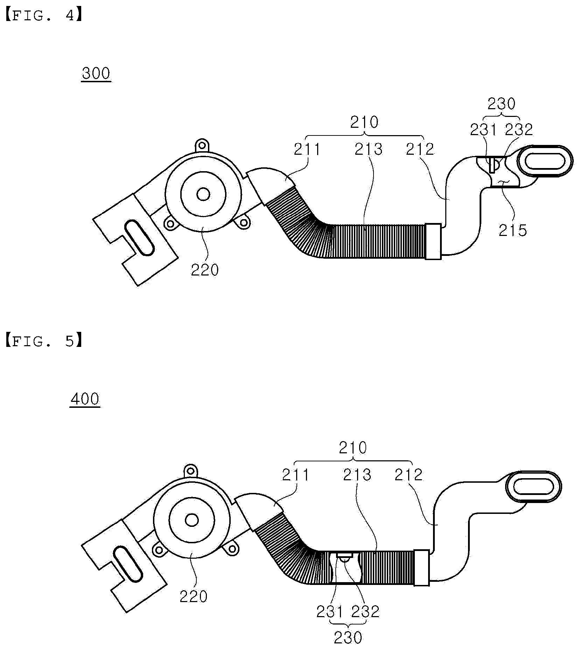

FIG. 4 is an exemplary view illustrating a duct module according to a second exemplary embodiment of the present invention.

A duct module 300 according to the second exemplary embodiment includes a duct 210, an air intake module 220, and a sterilization module 230.

In the duct module 300 of the present exemplary embodiment, the sterilization module 230 is fixed to an inner side surface of the second rigid portion 212 of the duct 210. In this case, the sterilization light source 232 may be disposed to face the outlet of the duct 210.

Accordingly, the sterilization module 230 irradiates sterilization light from the sterilization light source 232 to the air moving toward the outlet. That is, the air is continuously exposed to the sterilization light, while moving from the sterilization module 230 to the outlet.

Such disposition of the sterilization module 230 may increase time for air to be exposed to the sterilization light to improve sterilization efficiency.

FIG. 5 is an exemplary view illustrating a duct module according to a third exemplary embodiment of the present invention.

A duct module 400 according to the third exemplary embodiment includes a duct 210, an air intake module 220, and a sterilization module 230. The duct module 400 according to the third exemplary embodiment is different from the first exemplary embodiment in the position in which the sterilization module 230 is disposed.

According to the present exemplary embodiment, the sterilization module 230 is disposed on the flexible portion 213. At this time, the sterilization module 230 is fixed to the duct 210, while penetrating through a side surface of the duct 210.

For example, an insertion hole into which the sterilization module 230 is inserted may be formed on a side surface of the flexible portion 213. The sterilization module 230 is inserted in and fixed to the insertion hole of the flexible portion 213 so that the sterilization light source 232 is positioned inside the flexible portion 213. In this case, the substrate 231 of the sterilization module 230 may be fixed to the side surface of the flexible portion 213.

The sterilization module 230 may be combined with and separated from the duct 210 from the outside of the duct 210 through the insertion hole of the duct 210. Therefore, in the duct module 400 of the present exemplary embodiment, the sterilization module 230 may be replaced without disassembling the duct 210.

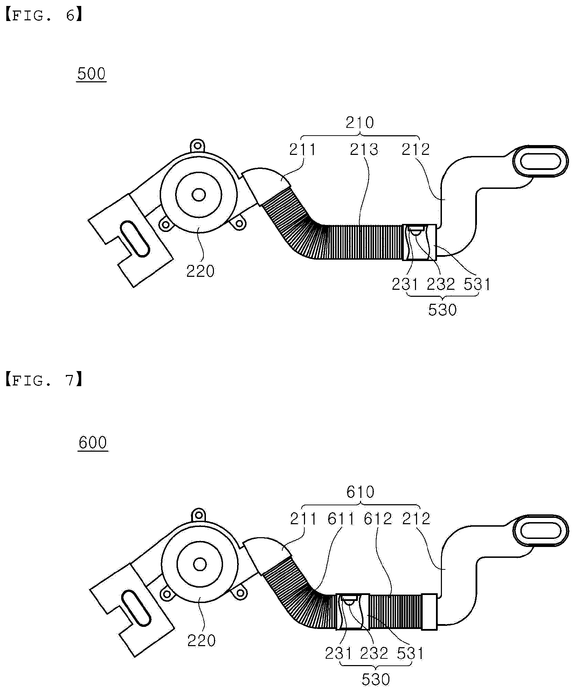

FIG. 6 is an exemplary view illustrating a duct module according to a fourth exemplary embodiment of the present invention.

The duct module 500 according to a fourth exemplary embodiment includes a duct 210, an air intake module 220, and a sterilization module 530.

In the present exemplary embodiment, the sterilization module 530 includes a substrate 231, a sterilization light source 232, and a sterilization module body 531.

The sterilization module body 531 has a tube structure having an internal space connected from one side to the other side. The sterilization module 530 is disposed in the internal space of the sterilization module body 531.

One side of the sterilization module body 531 is coupled to the flexible portion 213, and the other side is coupled to the second rigid portion 212. At this time, the internal space of the sterilization module body 531 also becomes part of the duct air passage through which air flows. The sterilization module body 531 is not coupled and fixed to the flexible portion 213 and the second rigid portion 212, but may be separated. For example, the sterilization module body 531, the flexible portion 213, and the second rigid portion 212 are threaded on the inner side surface or the outer surface, so that they may be coupled and separated by a screw coupling method. In addition, the sterilization module body 531 may be coupled to and separated from the flexible portion 213 and the second rigid portion 212 in various manners.

In the duct module 500 according to the present exemplary embodiment, the sterilization module body 531 may be separated from the duct 210, and thus, it is easy to replace the sterilization module 530 or the sterilization light source 232.

FIG. 7 is an exemplary view illustrating a duct module according to a fifth exemplary embodiment of the present invention.

The duct module 600 according to the fifth exemplary embodiment includes a duct 610, an air intake module 220, and a sterilization module 530.

In the present exemplary embodiment, the sterilization module 530 includes a substrate 231, a sterilization light source 232, and a sterilization module body 531. The sterilization module 530 is the same as the sterilization module 530 of FIG. 6.

In the present exemplary embodiment, the duct 610 includes a first rigid portion 211, a second rigid portion 212, a first flexible portion 611, and a second flexible portion 612.

The first flexible portion 611 and the second flexible portion 612 of the duct 610 are disposed between the first rigid portion 211 and the second rigid portion 212. Also, the sterilization module 530 is disposed between the first flexible portion 611 and the second flexible portion 612.

Accordingly, one side of the first flexible portion 611 is coupled to the first rigid portion 211, and the other side is coupled to one side of the sterilization module body 531. In addition, one side of the second flexible portion 612 is coupled to the other side of the sterilization module body 531, and the other side of the second flexible portion 612 is coupled to the second rigid portion 212.

The sterilization module 530 of the present exemplary embodiment may be coupled to and separated from the first flexible portion 611 and the second flexible portion 612. Accordingly, in the duct module 600 of the present exemplary embodiment, it is easy to replace the sterilization module 530.

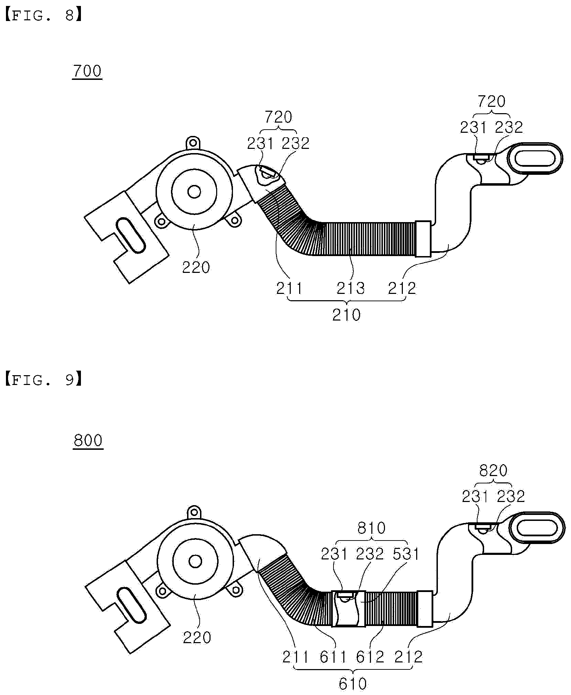

FIG. 8 is an exemplary view illustrating a duct module according to a sixth exemplary embodiment of the present invention.

A duct module 700 according to the sixth exemplary embodiment includes a plurality of sterilization modules. For example, the duct module 700 includes a first sterilization module 710 and a second sterilization module 720.

Referring to FIG. 8, the first sterilization module 710 and the second sterilization module 720 have the same structure. Both the first sterilization module 710 and the second sterilization module 720 include a substrate 231 and a sterilization light source 232 and are disposed inside the duct 210.

The first sterilization module 710 is disposed on an inner side surface of the first rigid portion 211. The first sterilization module 710 primarily sterilizes air discharged from the air intake module 220 and passing through the inside of the first rigid portion 211.

In addition, the second sterilization module 720 is disposed on an inner side surface of the second rigid portion 212. The second sterilization module 720 secondary sterilizes the primarily sterilized air that has been primarily sterilized and has passed through the flexible portion 213, before being discharged to the outside of the duct 210.

FIG. 9 is an exemplary view illustrating a duct module according to a seventh exemplary embodiment of the present invention.

A duct module 800 according to the seventh exemplary embodiment includes a plurality of sterilization modules. For example, the duct module 800 includes a first sterilization module 810 and a second sterilization module 820.

Referring to FIG. 9, the first sterilization module 810 and the second sterilization module 820 have different structures. The first sterilization module 810 includes a substrate 231, a sterilization light source 232, and a sterilization module body 531.

The first sterilization module 810 is disposed between the first flexible portion 611 and the second flexible portion 612. At this time, one side of the sterilization module body 531 of the first sterilization module 810 is coupled to the first flexible portion 611, and the other side thereof is coupled to the second flexible portion 612.

The first sterilization module 810 sterilizes air passing through the inside of the sterilization module body 531. That is, the first sterilization module 810 primarily sterilizes the air passing through the inside of the flexible portion.

The second sterilization module 820 is disposed on an inner side surface of the second rigid portion 212. The second sterilization module 820 sterilizes the air that has been first sterilized, while passing through the flexible portion, before being discharged to the outside of the duct 610.

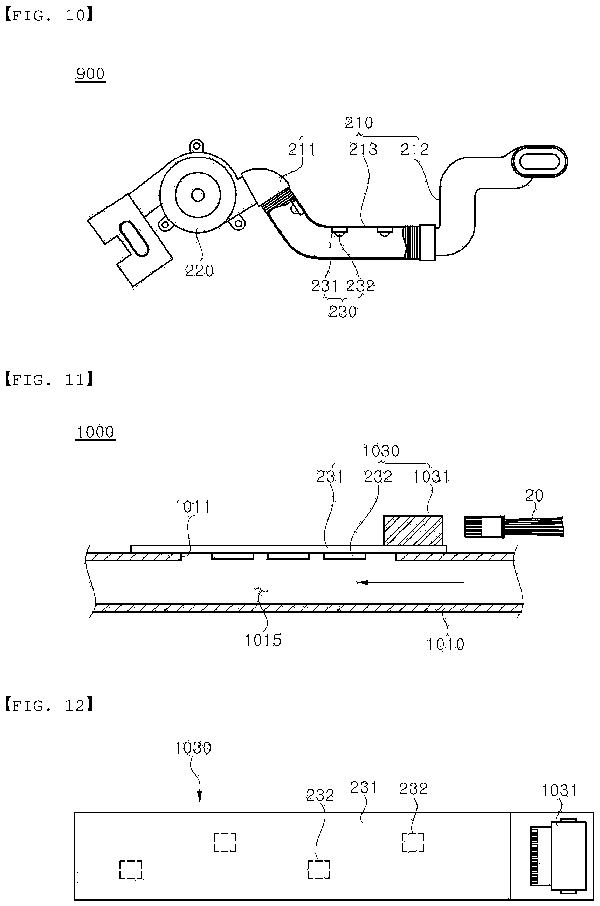

FIG. 10 is an exemplary view illustrating a duct module according to an eighth exemplary embodiment of the present invention.

A duct module 900 according to the eighth exemplary embodiment includes a plurality of sterilization modules 230.

Referring to FIG. 10, the plurality of sterilization modules 230 are disposed to be dispersed in the flexible portion 213 of the duct 210. Accordingly, air intaken through the air intake module 220 may be continuously exposed to sterilization light, while passing through the flexible portion 213 of the duct 210, to be sterilized.

In this way, the duct module 700 of the sixth exemplary embodiment to the duct module 900 of the eighth exemplary embodiment may include a plurality of sterilization modules to increase a sterilization time of the air flowing inside the duct. That is, the duct module of the present exemplary embodiment includes a plurality of sterilization modules to improve air sterilization efficiency.

In the present exemplary embodiment, it is described as an example that the duct module is mounted on the seat. However, the duct module of the present exemplary embodiment does not emit sterilization light to the outside of the duct, and since the structure of the duct is freely changed by the flexible portion, the duct module may be disposed anywhere inside the vehicle.

FIGS. 11 and 12 are exemplary views illustrating a duct module according to a ninth exemplary embodiment of the present invention.

FIGS. 11 and 12 show a structure in which a sterilization module 1030 is mounted on the duct 1010 in the structure of a duct module 1000 according to the ninth exemplary embodiment.

Referring to FIGS. 11 and 12, the duct module 1000 includes a sterilization module 1030, and the sterilization module 1030 includes a substrate 231, a plurality of sterilization light sources 232, and a connector 1031.

Referring to FIG. 11, a plurality of sterilization light sources 232 are disposed on one surface of the substrate 231, and a connector 1031 is disposed on the other surface of the substrate 231.

The plurality of sterilization light sources 232 may be variously disposed on the substrate 231. For example, as shown in FIG. 12, the plurality of sterilization light sources 232 may be disposed on the substrate 231 in a zigzag manner. Accordingly, it is possible to sufficiently irradiate sterilization light to the air moving near one side or the other side of the duct air passage 1015.

A sterilization module mounting hole 1011 having a through structure is formed in the duct 1010. The sterilization module 1030 is mounted in the sterilization module mounting hole 1011.

For example, the sterilization module mounting hole 1011 is formed to have a size smaller than that of the substrate 231. Accordingly, as shown in FIG. 11, in the sterilization module 1030, the sterilization light source 232 is inserted into the duct 1010 through the sterilization module mounting hole 1011. In this case, an edge portion of the substrate 231 spans on an outer surface of the duct 1010. Accordingly, while the sterilization module 1030 is mounted on the outside of the duct 1010, the sterilization light source 232 may irradiate light to the duct air passage 1015. The sterilization module 1030 may be fixed to the duct 1010 by applying an adhesive between one surface of the substrate 231 and the outer surface of the duct 1010. Alternatively, the sterilization module 1030 may be fixed to the duct 1010 by various methods such as a screw fastening method.

The connector 1031 is disposed on the other surface of the substrate 231 to be positioned outside the duct 1010. The connector 1031 and the sterilization light source 232 are electrically connected to each other by wiring formed on the substrate 231.

In the present exemplary embodiment, since the connector 1031 is located outside the duct 1010, it is easy for the connector 1031 to be connected to an electric wire 20 connected to an external power source. In addition, as in the present exemplary embodiment, when the sterilization module 1030 is mounted from the outside of the duct 1010, the sterilization module 1030 may be easily replaced.

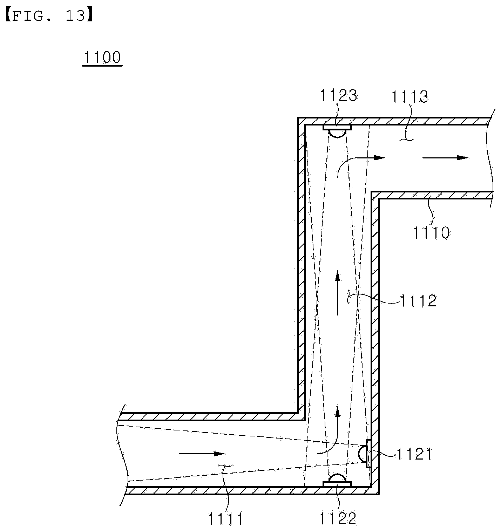

FIG. 13 is an exemplary view illustrating a duct module according to a tenth exemplary embodiment of the present invention.

As for a duct module 1100 of the present exemplary embodiment, a structure of the duct and a position of a sterilization module will be mainly described. For the description of omitted components, the exemplary embodiment of the duct module described above may be referred to.

A duct 1110 of the present exemplary embodiment may include at least one bent portion. Accordingly, air flowing in the duct air passage, which is an internal space of the duct 1110, may change in movement direction, while passing through the bent portion of the duct 1110. The bent portion of the duct 1110 may be located in any of a rigid portion and a flexible portion of the duct 1110.

For example, duct 1110 may include a plurality of duct air passages connected to each other. Also, at least one of the plurality of duct air passages may allow air to flow in a direction different from that of the other duct air passages. In this case, at least one of the duct air passages in which the air movement direction is changed may have a structure in which a length is longer than an inner diameter.

Referring to FIG. 13, the duct 1110 may include a first duct air passage 1111, a second duct air passage 1112, and a third duct air passage 1113.

Air flows in the same direction in the first duct air passage 1111 and the third duct air passage 1113. However, air movement direction in the second duct air passage 1112 is different from that in the first duct air passage 1111 and the third duct air passage 1113.

Here, the second duct air passage 1112 has a structure in which a length is longer than an inner diameter.

According to the present exemplary embodiment, the plurality of sterilization modules may be disposed at the bent portion of the duct 1110. That is, the plurality of sterilization modules may be installed on an inner wall of the second duct air passage 1112 or an inner wall of a portion adjacent to the second duct air passage 1112.

At least one of the plurality of sterilization modules may be arranged to have an irradiation distance greater than a diameter of the duct air passage.

For example, the first sterilization module 1121 to the third sterilization module 1123 may be disposed on an inner wall of the second duct air passage 1112.

The first sterilization module 1121 may be disposed on an inner wall of one end of the second duct air passage 1112 and may irradiate light toward the first duct air passage 1111. In this case, the first sterilization module 1121 may irradiate light in a direction opposite to the movement direction of the air flowing through the first duct air passage 1111.

The second sterilization module 1122 may be disposed on an inner wall of one end of the second duct air passage 1112 to irradiate light in a longitudinal direction of the second duct air passage 1112. Here, one end of the second duct air passage 1112 is a portion in which a movement direction of the air passing through the first duct air passage 1111 is changed.

In addition, the third sterilization module 1123 may be disposed on an inner wall of the other end of the second duct air passage 1112 to irradiate light in a longitudinal direction of the second duct air passage 1112. Here, the other end of the second duct air passage 1112 is a portion in which a movement direction of the air passing through the second duct air passage 1112 is changed.

The second sterilization module 1122 and the third sterilization module 1123 may be disposed to face each other. Here, the second sterilization module 1122 irradiates light in the air movement direction of the second duct air passage 1112, and the third sterilization module 1123 irradiates light in a direction opposite to the air movement direction of the second duct air passage 1112.

As such, the duct module 1100 of the present exemplary embodiment may have an irradiation distance longer than the length of the second duct air passage 1112 and may include a second sterilization module 1122 and a third sterilization module 1123 disposed to face each other in the longitudinal direction of the second duct air passage 1112. Accordingly, the duct module 1100 of the present exemplary embodiment increases time during which the air passing through the second duct air passage 1112 is exposed to the sterilization light, thereby improving sterilization efficiency.

In the description of the present exemplary embodiment, the duct 1110 has a structure including the first duct air passage 1111 to the third duct air passage 1113 by including two bent portions, but the structure of the duct module 1100 is not limited thereto.

In addition, in the description of the present exemplary embodiment, the duct module 1100 includes three sterilization modules, but the number and position of the sterilization modules installed in the duct 1110 may be changed according to selection of those skilled in the art.

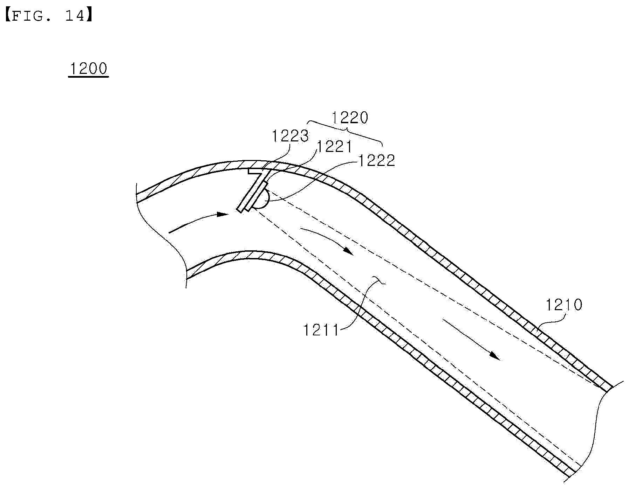

FIG. 14 is an exemplary view illustrating a duct module according to an eleventh exemplary embodiment of the present invention.

As for a duct module 1200 of the present exemplary embodiment, a structure of a duct and a position of a sterilization module will be mainly described. For the description of the omitted components, the exemplary embodiment of the duct module described above may be referred to.

The duct module 1200 according to the eleventh exemplary embodiment includes a duct 1210 in which at least a portion is bent.

Referring to FIG. 14, the duct 1210 may include a gently bent portion.

In addition, the sterilization module 1220 may be mounted on an inner wall of the gently curved portion of the duct 1210.

The sterilization module 1220 may be mounted to have an inclination on an inner wall of the duct air passage 1211.

For example, the sterilization module 1220 may include a support member 1223, and a substrate 1221 on which a sterilization light source 1222 is mounted may be disposed to be inclined on an inner wall of the duct air passage 1211 through the support member 1223. Here, the support member 1223 may be mounted on an inner wall of the duct air passage 1211 to have an angle at which sterilization light is irradiated in the movement direction of air or in a direction opposite to the air movement direction when the sterilization module 1220 is mounted.

Alternatively, the sterilization module 1220 may be mounted on an inner wall of the duct air passage 1211 so that sterilization light may be irradiated at a specific angle through the support member 1223. For example, the sterilization module 1220 may be arranged such that sterilization light is minimally irradiated to the inner wall of the duct air passage 1211.

According to the present exemplary embodiment, the support member 1223 may be formed to be adjusted in angle in consideration of a diameter of the duct air passage 1211 and a sterilization light irradiation angle of the sterilization module 1220.

For example, the support member 1223 may include a mounting portion mounted on the inner wall of the duct air passage 1211 and an installation portion on which the sterilization module 1220 is installed. In this case, the installation portion may be connected to the mounting portion so that an angle may be adjusted. Accordingly, the sterilization module 1220 may adjust an irradiation angle of the sterilization light by adjusting the angle of the installation portion of the support member 1223.

In the present exemplary embodiment, the sterilization module 1220 includes a separate support member 1223, but the substrate 1221 may replace a role of the support member 1223.

In the duct module 1200 according to the present exemplary embodiment, the sterilization module 1220 may be disposed to be inclined at a specific angle so that sterilization light may be irradiated in the movement direction of air or the opposite direction, and at the same time, is minimally irradiated on the inner wall of the duct air passage 1211.

Therefore, the duct module 1200 according to the present exemplary embodiment may irradiate sterilization light to air as much as possible, thereby improving the sterilization efficiency without changes such as addition of the sterilization light source 1222, air movement speed control, and air amount control.

As described above, the detailed description of the present invention has been made by the exemplary embodiments with reference to the accompanying drawings, but since the exemplary embodiments described above have only been described with preferred examples of the present invention, the present invention should not be limited only to the above exemplary embodiments the scope of the present invention should be understood as the following claims and their equivalents.

## Claims

1. A duct module (200) comprising:
a duct (210) having a duct air passage (215) therein and including a first rigid portion (211), a second rigid portion (212), and a flexible portion (213) disposed between the first rigid portion (211) and the second rigid portion (212);
an air intake module (220) connected to the first rigid portion (211) of the duct (210) and guiding external air to the duct air passage(215) ; and
a sterilization module (230) disposed in the duct (210) and emitting sterilization light to the duct air passage (215),
wherein the duct air passage (215) is formed as an internal space of the first rigid portion (211), the second rigid portion (212), and the flexible portion (213), and
wherein the sterilization module (230) is mounted from an outside of the duct (210) such to be replaced without disassembling the duct (210).

2. The duct module (200) of claim 1, wherein
the sterilization module (230) includes:
a substrate (231); and
a sterilization light source (232) mounted on the substrate (231) and emitting an ultraviolet light.

3. The duct module (200) of claim 2, wherein the sterilization module (230) is disposed on an inner side surface of the second rigid portion of the duct (210).

4. The duct module (200) of claim 2, wherein the sterilization module (230) is disposed to penetrate through a side surface of the duct (210).

5. The duct module (200) of claim 2, wherein
the sterilization module (230) further includes a sterilization module body (531) having one side and the other side open and having an internal space connecting the one side and the other side, and
the substrate (231) and the sterilization light source (232) are disposed in the internal space of the sterilization module body (531).

6. The duct module (200) of claim 5, wherein the one side of the sterilization module body (532) is connected to the flexible portion (213), and the other side is connected to the second rigid portion (212).

7. The duct module (200) of claim 5, wherein
the flexible portion (213) includes a first flexible portion (611) connected to the first rigid portion (211) and a second flexible portion (612) connected to the second rigid portion (212), and
the one side of the sterilization module body (531) is connected to the first flexible portion (611), and the other side is connected to the second flexible portion (612).

8. The duct module (200) of claim 1, wherein the flexible portion (213) of the duct has a variable length.

9. The duct module (200) of claim 1, wherein the air intake module (220) includes a fan, and an air outlet of the fan is connected to the first rigid portion (211) of the duct (210).

10. A duct module (300) comprising
a duct (210) having a duct air passage (215) therein and including a first rigid portion (211), a second rigid portion (212), and a flexible portion (213) disposed between the first rigid portion (211) and the second rigid portion (212);
an air intake module (220) connected to the first rigid portion (211) of the duct (210) and guiding air to the duct air passage (215); and
a sterilization module (230) disposed in the duct (210) and including a sterilization light source (232) to discharge sterilization light into the duct air passage (215),
wherein the duct air passage (215) is formed as an internal space of the first rigid portion (211), the second rigid portion (212), and the flexible portion (213),
wherein the sterilization module (230) is disposed on an inner side surface of the second rigid portion (212) of the duct (210), and
wherein the sterilization light source (232) is disposed to face an outlet of the duct (210), and irradiates sterilization light from the sterilization light source (232) to the air moving toward the outlet.

11. The duct module (300) of claim 10, wherein the seat air passage (215) is connected to the second rigid portion (212) of the duct (210).

12. The duct module (300) of claim 10, wherein one end of the second rigid portion (212) is connected to the flexible portion (213).

13. The duct module (300) of claim 12, wherein the sterilization module (230) is separated from the duct (210) from an outside of the duct (210) through an insertion hole of the duct (210).

14. The duct module (300) of claim 12, wherein the sterilization module (230) is disposed to penetrate through a side surface of the duct (210).

15. The duct module (300) of claim 12, wherein
the sterilization module (230) further includes a sterilization module body (531) having one side and the other side open and having an internal space connecting the one side and the other side, and
the substrate (231) and the sterilization light source (23) are disposed in the internal space of the sterilization module body (531).

## Patentansprüche

1. Kanalmodul (200), umfassend:
einen Kanal (210), der in seinem Inneren einen Kanalluftdurchgang (215) aufweist und einen ersten starren Abschnitt (211), einen zweiten starren Abschnitt (212) und einen flexiblen Abschnitt (213), der zwischen dem ersten starren Abschnitt (211) und dem zweiten starren Abschnitt (212) angeordnet ist, aufweist;
ein Lufteinlassmodul (220), das mit dem ersten starren Abschnitt (211) des Kanals (210) verbunden ist und Außenluft zu dem Kanalluftdurchgang (215) führt; und
ein Sterilisationsmodul (230), das in dem Kanal (210) angeordnet ist und Sterilisationslicht zu dem Kanalluftdurchgang (215) emittiert,
wobei der Kanalluftdurchgang (215) als ein Innenraum des ersten starren Abschnitts (211), des zweiten starren Abschnitts (212) und des flexiblen Abschnitts (213) gebildet ist, und
wobei das Sterilisationsmodul (230) von außerhalb des Kanals (210) so montiert ist, dass es ohne Demontage des Kanals (210) ausgetauscht werden kann.

2. Kanalmodul (200) nach Anspruch 1, wobei das Sterilisationsmodul (230) aufweist:
ein Substrat (231); und
eine Sterilisationslichtquelle (232), die auf dem Substrat (231) montiert ist und ein ultraviolettes Licht emittiert.

3. Kanalmodul (200) nach Anspruch 2, wobei das Sterilisationsmodul (230) an einer Innenseitenfläche des zweiten starren Abschnitts des Kanals (210) angeordnet ist.

4. Kanalmodul (200) nach Anspruch 2, wobei das Sterilisationsmodul (230) so angeordnet ist, dass es eine Seitenfläche des Kanals (210) durchdringt.

5. Kanalmodul (200) nach Anspruch 2, wobei
das Sterilisationsmodul (230) des Weiteren einen Sterilisationsmodulkörper (531) aufweist, der eine Seite und die andere Seite offen hat und einen Innenraum aufweist, der die eine Seite und die andere Seite verbindet, und
das Substrat (231) und die Sterilisationslichtquelle (232) in dem Innenraum des Sterilisationsmodulkörpers (531) angeordnet sind.

6. Kanalmodul (200) nach Anspruch 5, wobei die eine Seite des Sterilisationsmodulkörpers (532) mit dem flexiblen Abschnitt (213) verbunden ist und die andere Seite mit dem zweiten starren Abschnitt (212) verbunden ist.

7. Kanalmodul (200) nach Anspruch 5, wobei
der flexible Abschnitt (213) einen ersten flexiblen Abschnitt (611), der mit dem ersten starren Abschnitt (211) verbunden ist, und einen zweiten flexiblen Abschnitt (612), der mit dem zweiten starren Abschnitt (212) verbunden ist, aufweist, und
die eine Seite des Sterilisationsmodulkörpers (531) mit dem ersten flexiblen Abschnitt (611) verbunden ist und die andere Seite mit dem zweiten flexiblen Abschnitt (612) verbunden ist.

8. Kanalmodul (200) nach Anspruch 1, wobei der flexible Abschnitt (213) des Kanals eine variable Länge aufweist.

9. Kanalmodul (200) nach Anspruch 1, wobei das Lufteinlassmodul (220) ein Gebläse aufweist und ein Luftauslass des Gebläses mit dem ersten starren Abschnitt (211) des Kanals (210) verbunden ist.

10. Kanalmodul (300), umfassend
einen Kanal (210), der in seinem Inneren einen Kanalluftdurchgang (215) aufweist und einen ersten starren Abschnitt (211), einen zweiten starren Abschnitt (212) und einen flexiblen Abschnitt (213), der zwischen dem ersten starren Abschnitt (211) und dem zweiten starren Abschnitt (212) angeordnet ist, aufweist;
ein Lufteinlassmodul (220), das mit dem ersten starren Abschnitt (211) des Kanals (210) verbunden ist und Luft zu dem Kanalluftdurchgang (215) führt; und
ein Sterilisationsmodul (230), das in dem Kanal (210) angeordnet ist und eine Sterilisationslichtquelle (232) aufweist, um Sterilisationslicht in den Kanalluftdurchgang (215) abzugeben,
wobei der Kanalluftdurchgang (215) als ein Innenraum des ersten starren Abschnitts (211), des zweiten starren Abschnitts (212) und des flexiblen Abschnitts (213) gebildet ist,
wobei das Sterilisationsmodul (230) an einer Innenseitenfläche des zweiten starren Abschnitts (212) des Kanals (210) angeordnet ist, und
wobei die Sterilisationslichtquelle (232) so angeordnet ist, dass sie einem Auslass des Kanals (210) zugewandt ist, und Sterilisationslicht von der Sterilisationslichtquelle (232) zu der Luft abstrahlt, die sich in Richtung des Auslasses bewegt.

11. Kanalmodul (300) nach Anspruch 10, wobei der Sitzluftdurchgang (215) mit dem zweiten starren Abschnitt (212) des Kanals (210) verbunden ist.

12. Kanalmodul (300) nach Anspruch 10, wobei ein Ende des zweiten starren Abschnitts (212) mit dem flexiblen Abschnitt (213) verbunden ist.

13. Kanalmodul (300) nach Anspruch 12, wobei das Sterilisationsmodul (230) von dem Kanal (210) von einer Außenseite des Kanals (210) durch ein Einführloch des Kanals (210) getrennt ist.

14. Kanalmodul (300) nach Anspruch 12, wobei das Sterilisationsmodul (230) so angeordnet ist, dass es eine Seitenfläche des Kanals (210) durchdringt.

15. Kanalmodul (300) nach Anspruch 12, wobei das Sterilisationsmodul (230) des Weiteren einen Sterilisationsmodulkörper (531) aufweist, der eine Seite und die andere Seite offen hat und einen Innenraum aufweist, der die eine Seite und die andere Seite verbindet, und
das Substrat (231) und die Sterilisationslichtquelle (23) in dem Innenraum des Sterilisationsmodulkörpers (531) angeordnet sind.

## Revendications

1. Module de conduit (200) comprenant :
un conduit (210) possédant un passage d'air de conduit (215) dans celui-ci et comportant une première partie rigide (211), une seconde partie rigide (212), et une partie flexible (213) disposée entre la première partie rigide (211) et la seconde partie rigide (212) ;
un module d'admission d'air (220) relié à la première partie rigide (211) du conduit (210) et guidant l'air extérieur vers le passage d'air de conduit (215) ; et
un module de stérilisation (230) disposé dans le conduit 210) et émettant une lumière de stérilisation dans le passage d'air de conduit (215),
dans lequel le passage d'air de conduit (215) est formé comme un espace interne de la première partie rigide (211), de la seconde partie rigide (212), et de la partie flexible (213), et
dans lequel le module de stérilisation (230) est monté à partir d'un extérieur du conduit (210) de manière à pouvoir être remplacé sans démonter le conduit (210).

2. Module de conduit (200) selon la revendication 1, dans lequel le module de stérilisation (230) comporte :
un substrat (231) ; et
une source de lumière de stérilisation (232) montée sur le substrat (231) et émettant une lumière ultraviolette.

3. Module de conduit (200) selon la revendication 2, dans lequel le module de stérilisation (230) est disposé sur une surface latérale intérieure de la seconde partie rigide du conduit (210).

4. Module de conduit (200) selon la revendication 2, dans lequel le module de stérilisation (230) est disposé pour pénétrer à travers une surface latérale du conduit (210).

5. Module de conduit (200) selon la revendication 2, dans lequel
le module de stérilisation (230) comporte en outre un corps de module de stérilisation (531) ayant un côté et l'autre côté ouverts et ayant un espace interne reliant ledit un côté et l'autre côté, et
le substrat (231) et la source de lumière de stérilisation (232) sont disposés dans l'espace interne du corps du module de stérilisation (531) .

6. Module de conduit (200) selon la revendication 5, dans lequel ledit un côté du corps du module de stérilisation (532) est relié à la partie flexible (213), et l'autre côté est relié à la seconde partie rigide (212).

7. Module de conduit (200) selon la revendication 5, dans lequel
la partie flexible (213) comporte une première partie flexible (611) reliée à la première partie rigide (211) et une seconde partie flexible (612) reliée à la seconde partie rigide (212), et
ledit un côté du corps du module de stérilisation (531) est relié à la première partie flexible (611), et l'autre côté est relié à la seconde partie flexible (612) .

8. Module de conduit (200) selon la revendication 1, dans lequel la partie flexible (213) du conduit a une longueur variable.

9. Module de conduit (200) selon la revendication 1, dans lequel le module d'admission d'air (220) comporte un ventilateur, et une sortie d'air du ventilateur est reliée à la première partie rigide (211) du conduit (210).

10. Module de conduit (300) comprenant
un conduit (210) possédant un passage d'air de conduit (215) dans celui-ci et comportant une première partie rigide (211), une seconde partie rigide (212), et une partie flexible (213) disposée entre la première partie rigide (211) et la seconde partie rigide (212) ;
un module d'admission d'air (220) relié à la première partie rigide (211) du conduit (210) et guidant l'air vers le passage d'air de conduit (215) ; et
un module de stérilisation (230) disposé dans le conduit (210) et comportant une source de lumière de stérilisation (232) pour décharger la lumière de stérilisation dans le passage d'air de conduit (215),
dans lequel le passage d'air de conduit (215) est formé comme un espace interne de la première partie rigide (211), de la seconde partie rigide (212), et de la partie flexible (213),
dans lequel le module de stérilisation (230) est disposé sur une surface latérale intérieure de la seconde partie rigide (212) du conduit (210), et
dans lequel la source de lumière de stérilisation (232) est disposée pour faire face à une sortie du conduit (210), et irradie la lumière de stérilisation de la source de lumière de stérilisation (232) vers l'air se déplaçant vers la sortie.

11. Module de conduit (300) selon la revendication 10, dans lequel le passage d'air de siège (215) est relié à la seconde partie rigide (212) du conduit (210).

12. Module de conduit (300) selon la revendication 10, dans lequel une extrémité de la seconde partie rigide (212) est reliée à la partie flexible (213).

13. Module de conduit (300) selon la revendication 12, dans lequel le module de stérilisation (230) est séparé du conduit (210) depuis un extérieur du conduit (210) à travers un trou d'insertion du conduit (210).

14. Module de conduit (300) selon la revendication 12, dans lequel le module de stérilisation (230) est disposé pour pénétrer à travers une surface latérale du conduit (210).

15. Module de conduit (300) selon la revendication 12, dans lequel
le module de stérilisation (230) comporte en outre un corps de module de stérilisation (531) ayant un côté et l'autre côté ouverts et ayant un espace interne reliant ledit un côté et l'autre côté, et
le substrat (231) et la source de lumière de stérilisation (23) sont disposés dans l'espace interne du corps du module de stérilisation (531) .
